(19) Europäisches Patentamt
European Patent Office
Office européen des brevets

(11) EP 2 799 534 A1

(12) **DEMANDE DE BREVET EUROPEEN**

(43) Date de publication:
**05.11.2014 Bulletin 2014/45**

(21) Numéro de dépôt: **13166282.7**

(22) Date de dépôt: **02.05.2013**

(51) Int Cl.:
*C12M 1/107* (2006.01)    *C12M 1/00* (2006.01)
*C12P 5/02* (2006.01)    *C12M 1/36* (2006.01)

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(71) Demandeur: **Greenwatt SA**
**1348 Louvain-la-Neuve (BE)**

(72) Inventeurs:
• **THOMAS, Morgan**
**1495 Villes-la-Ville (BE)**
• **SCHMITT, Matthieu**
**1435 Mont-Saint-Guibert (BE)**

(74) Mandataire: **De Groote, Christophe et al**
**Pecher & de Groote sprl**
**Centre Monnet**
**Avenue Jean Monnet, 1**
**1348 Louvain-la-Neuve (BE)**

(54) **Procédé et appareil de digestion de matière organique**

(57) L'invention concerne un procédé de méthanisation de matière organique en deux étapes dans lequel les paramètres de chacune des étapes sont contrôlés indépendamment les uns des autres afin d'obtenir un rendement global de réaction satisfaisant en fonction de la nature de la matière organique utilisée lors du procédé de méthanisation. L'invention concerne également un appareil pour mettre en oeuvre le procédé selon l'invention.

Fig. 1

EP 2 799 534 A1

**Description**

**Domaine de l'invention**

L'invention se rapporte au domaine de la digestion de matière organique. Plus particulièrement, l'invention se rapporte à un procédé en deux étapes permettant la production de biogaz à partir de matière organique au moins en partie solide. L'invention se rapporte également à un appareil pour mettre en oeuvre un procédé de digestion de matière organique selon l'invention.

**État de la technique**

La digestion de matière organique en vue de produire des biogaz est un procédé utilisé depuis de nombreuses années. Ainsi, des procédés de méthanisation de matière organique, sous forme liquide ou sous forme solide, afin de produire du biogaz riche en méthane sont employés pour transformer ces matières organiques. La méthanisation est donc un procédé connu. De façon générale, la méthanisation est un processus se déroulant en plusieurs phases. La première phase consiste à dégrader la matière organique. Cette phase se déroule sous la forme d'une hydrolyse de la matière organique. La phase suivante est une phase d'acidogenèse, consistant en la formation d'acides organiques, notamment des acides gras volatils. Une troisième phase d'acétogenèse consiste notamment en la formation d'acétates et d'acide formique à partir notamment d'acides organiques. Enfin, la dernière phase d'un procédé classique de méthanisation est la phase dite de méthanogenèse, transformant au moins une partie des acétates notamment en biogaz, comprenant notamment du méthane.

Ces différentes phases requièrent des conditions de réaction différentes afin que les cinétiques de réaction soient satisfaisantes. Ainsi, le procédé de méthanisation est couramment séparé en deux étapes : une première étape regroupe la phase d'hydrolyse et la phase d'acidogenèse ; une seconde étape regroupe la phase d'acétogenèse et la phase de méthanogenèse. Lors d'une méthanisation en deux étapes, chacune des étapes est réalisée dans un compartiment qui lui est propre (généralement dans deux cuves séparées). Avec ce procédé, il est possible de gérer la cinétique générale du procédé de méthanisation en jouant sur une des cinétiques d'une des étapes afin de réduire la durée du procédé, ou de produire une quantité plus importante de biogaz riche en méthane, ou un biogaz de plus grande qualité.

Généralement, la matière organique solide utilisée dans les procédés de méthanisation est composée d'une fraction rapidement fermentescible et d'une fraction plus récalcitrante. Lors de l'étape d'hydrolyse la fraction rapidement fermentescible passe rapidement à l'état hydrosoluble contrairement à la fraction récalcitrante qui requiert un temps beaucoup plus long. La matière organique se présente après l'étape d'hydrolyse sous deux formes : une forme hydrosoluble contenue majoritairement dans la phase liquide et une forme insoluble contenue majoritairement dans la phase solide. C'est pourquoi il est nécessaire de prévoir un traitement différent de la matière organique solide et de la matière organique liquide après la première étape d'hydrolyse. En effet, la matière organique solide nécessite un temps d'hydrolyse très variable en fonction de la taille de la matière organique et de la composition de cette matière organique en composés lentement dégradables comme par exemple la lignine, la cellulose ou l'hémicellulose. Cette nécessité demande alors de prévoir des appareils et des procédés traitant différemment la matière organique solide et la matière organique liquide.

On connaît des procédés permettant de modifier le temps de traitement d'un type de matière organique par rapport à l'autre. Par exemple, dans le document FR2920761, le temps de séjour de la matière organique liquide dans la cuve de réaction de la première étape est dissocié du temps de séjour de la matière solide dans la cuve de réaction de la première étape, grâce à la présence d'une étape de séparation de la phase liquide et de la phase solide après la première étape, permettant d'augmenter le temps passé par la matière solide dans la première cuve sans forcément impacter sur le temps de séjour de la matière liquide dans cette même cuve, grâce à un retour de la phase solide séparée dans la première cuve, et une modulation d'un flux de matière organique liquide entre le deuxième espace de réaction et le premier espace de réaction.

Cependant, un tel procédé n'est pas satisfaisant lorsque la matière organique solide a un temps de dégradation relativement long, obligeant à limiter l'apport de matière organique dans la première cuve, et ainsi réduire la quantité de biogaz produite pour une période de temps donné, ou à augmenter considérablement la taille de la première cuve dans laquelle se déroule notamment la phase d'hydrolyse.

Il manque donc un procédé, et incidemment un appareil pour mettre en oeuvre un tel procédé, permettant d'avoir un meilleur rendement en optimisant les temps de traitement.

**Résumé de l'invention**

L'invention est définie par les revendications indépendantes. Les revendications dépendantes définissent des modes de réalisation préférés de l'invention.

Selon l'invention il est fourni un procédé de méthanisation d'une matière organique au moins en partie solide comprenant les étapes de :

- introduction de ladite matière organique dans un premier espace de réaction,
- hydrolyse d'au moins une partie de la matière organique solide et acidogenèse de la matière organique au sein dudit premier espace de réaction,
- sortie de la matière organique du premier espace de réaction par un premier moyen d'extraction,
- introduction d'au moins une partie de la matière organique sortie du premier espace de réaction dans un second espace de réaction,
- acétogenèse et méthanogenèse de la matière organique présente au sein du second espace de réaction,
- sortie d'un premier digestat du second espace de réaction par un second moyen d'extraction,

caractérisé en ce que l'étape de sortie de la matière organique du premier espace de réaction est activée en fonction d'un degré d'hydrolyse de la partie solide de la matière organique introduite au sein dudit premier espace de réaction, et en ce qu'après la sortie de matière organique du premier espace de réaction, aucune partie solide de la matière organique extraite n'est réintroduite dans ledit premier espace de réaction.

Grâce à la méthode selon l'invention, le temps de séjour de la matière organique dans le premier espace de réaction est contrôlé en fonction d'un degré d'hydrolyse, permettant d'ajuster ce temps de séjour en fonction des caractéristiques intrinsèques de la matière organique, tout en conservant un procédé simple. Avec un tel procédé, la taille des espaces de réaction peut être de taille relativement modeste, en fonction des caractéristiques intrinsèques de la matière organique solide, et du volume de matière organique solide introduit dans le premier espace de réaction, sans impacter sur le rendement des réactions ultérieures d'un procédé de méthanisation.

Un autre but de l'invention est d'améliorer le traitement des différents types de matière organique présents. Ce but est atteint en séparant le traitement de la matière organique solide et de la matière organique liquide après la première étape d'hydrolyse et d'acidogenèse, afin d'optimiser le procédé de méthanisation, et notamment les étapes d'acétogenèse et de méthanogenèse, pour chaque forme de matière organique. Ainsi, de préférence, le procédé de méthanisation selon l'invention comporte :

- l'étape de sortie de la matière organique du premier espace de réaction est suivie d'une étape de séparation afin d'obtenir une matière organique liquide séparée et une matière organique solide séparée,
- ladite étape de séparation étant suivie d'une introduction de la matière organique liquide séparée dans le deuxième espace de réaction,
- une acétogenèse et une méthanogenèse de la matière organique liquide séparée est réalisée dans le second espace de réaction,
- la matière organique solide séparée est évacuée par une sortie pour matière organique solide séparée.

Afin de résoudre, au moins partiellement, les problèmes présents dans l'art antérieur, on fournit ainsi un procédé de traitement de matière organique au moins en partie solide dont au moins une phase du procédé est modulée indépendamment pour la matière organique solide et pour la matière organique liquide après la première étape d'hydrolyse et d'acidogenèse, tout en conservant un rendement d'autres étapes du procédé satisfaisant et sans avoir besoin de posséder des espaces de réaction de taille disproportionnée.

Selon ce mode de réalisation préféré, la matière organique liquide est séparée de la matière organique solide après la première étape d'hydrolyse et d'acidogenèse, et les conditions du traitement ultérieur de la matière organique liquide séparée peuvent être déterminées en fonction de la nature de la matière organique liquide séparée, sans avoir à prendre en compte les limitations que pourrait apporter la présence de matière organique solide, permettant d'avoir un temps de traitement optimisé de la matière organique liquide séparée en modulant indépendamment le temps de séjour de la matière organique liquide séparée au sein du second espace de réaction.

De manière plus préférée, le procédé selon l'invention comprend en outre les étapes suivantes :

- l'étape d'évacuation de la matière organique solide séparée est suivie de l'introduction de ladite matière organique solide séparée dans un troisième espace de réaction,
- une hydrolyse, une acidogenèse, une acétogenèse et une méthanogenèse de la matière organique solide séparée est réalisée dans le troisième espace de réaction,
- un second digestat est sorti du troisième espace de réaction par un troisième moyen d'extraction.

Selon ce mode préférentiel de réalisation, la matière organique solide séparée est traitée, et les étapes de traitement de la matière organique solide séparée et de la matière organique liquide séparée sont réalisées dans deux espaces différents, permettant d'obtenir un meilleur rendement pour chacun des types de matière organique, indépendamment l'une de l'autre. Ainsi, chacun des temps de séjour de la matière organique solide séparée et de la matière organique liquide séparée peuvent être définis indépendamment l'un de l'autre.

Dans un mode encore plus préféré, le procédé selon l'invention comprend une étape où la sortie du digestat d'un espace de réaction est activée en fonction d'un taux d'abattement d'une matière organique séparée présente dans ledit espace de réaction.

En effet, selon ce mode de réalisation, les étapes de sorties d'un digestat sont mieux contrôlées en fonction du rendement voulu par l'utilisateur du procédé, permettant d'ajuster le procédé aux besoins logistiques et/ou matériels en fonction de la taille de l'installation au sein de laquelle le procédé est réalisé, et de la nature et de la quantité de matière organique que l'utilisateur souhaite traiter.

L'invention concerne également un appareil de méthanisation permettant de mettre en oeuvre une méthode selon l'invention.

## Brève description des figures

Ces aspects ainsi que d'autres aspects de l'invention seront clarifiés dans la description détaillée de modes de réalisation particuliers de l'invention, référence étant faite aux dessins des figures, dans lesquelles :

Fig. 1    montre de manière schématique un appareil de méthanisation (50) selon un mode de réalisation de l'invention.

Fig. 2    montre de manière schématique un appareil de méthanisation (50) selon un mode de réalisation préféré de l'invention.

Fig. 3    montre de manière schématique un appareil de méthanisation (50) selon un mode de réalisation encore plus préféré de l'invention.

Fig. 4    montre de manière schématique un appareil de méthanisation (50) selon un mode de réalisation encore plus préféré de l'invention.

Fig. 5    montre de manière schématique un appareil de méthanisation (50) selon un mode de réalisation encore plus préféré de l'invention.

Les dessins des figures ne sont ni à l'échelle, ni proportionnés. Généralement, des éléments semblables sont dénotés par des références semblables dans les figures.

## Description détaillée de modes de réalisation de l'invention

Le procédé de méthanisation d'une matière organique au moins en partie solide selon l'invention comprend les étapes de :

- introduction de ladite matière organique dans un premier espace de réaction,
- hydrolyse d'au moins une partie de la matière organique solide et acidogenèse de la matière organique au sein dudit premier espace de réaction,
- sortie de la matière organique du premier espace de réaction par un premier moyen d'extraction,
- introduction d'au moins une partie de la matière organique sortie du premier espace de réaction dans un second espace de réaction,
- acétogenèse et méthanogenèse de la matière organique présente au sein du second espace de réaction,
- sortie d'un premier digestat du second espace de réaction par un second moyen d'extraction,

caractérisé en ce que l'étape de sortie de la matière organique du premier espace de réaction est activée en fonction d'un degré d'hydrolyse de la partie solide de la matière organique introduite au sein dudit premier espace de réaction, et en ce qu'après la sortie de matière organique du premier espace de réaction, aucune partie solide de la matière organique extraite n'est réintroduite dans ledit premier espace de réaction.

La matière organique comprend une matière organique biodégradable, et une matière organique résiduelle, ou non-biodégradable. Il s'entend que tout au long du procédé, la matière organique est constituée de matière organique biodégradable et de matière organique non biodégradable, ladite matière organique non biodégradable constituant la majorité de la matière organique en sortie de procédé, sous la forme d'un digestat. La matière organique est au moins en partie solide, ce qui signifie qu'elle peut se présenter sous la forme d'un mélange entre une matière organique solide et une matière organique liquide, ou sous la forme de matière organique solide uniquement. Par exemple, la matière organique introduite peut se présenter sous la forme de déchets de l'industrie agro-alimentaire, tels que des restes de légumes ou de fruits par exemple, pouvant être complémentée avec des liquides, comme par exemple, des eaux de lavage de l'industrie agro-alimentaire, des jus de pressage, du sang, du lait, ou tout liquide chargé en matière organique.

Le premier espace de réaction peut être une cuve de traitement ou un réacteur dans lequel la matière organique subit au moins une hydrolyse et une acidogenèse de façon à transformer au moins une partie de la matière organique en acides organiques.

La sortie de matière organique du premier espace de réaction est une étape lors de laquelle au moins une partie de la matière organique présente dans le premier espace de réaction est sortie de cet espace. Le premier moyen d'extraction peut être tout moyen permettant de sortir la matière organique, tel qu'un moyen par débordement, une vanne ayant au moins une position ouverte permettant le passage de matière organique et une position fermée empêchant le passage de matière organique, un moyen d'aspiration de la matière organique comme une pompe, un moyen de sortie de la matière organique par gravité, un tuyau comprenant un clapet avec une position ouverte et une position fermée.

Le second espace de réaction peut être tout espace permettant qu'une acétogenèse et qu'une méthanogenèse de la matière organique présente au sein du second espace puisse être réalisée de façon à transformer la matière organique présente au moins en partie en biogaz. Le biogaz produit durant la méthanogenèse peut être évacué par un moyen dédié, tel qu'une sortie pour biogaz via une canalisation en inox, en polyéthylène haute densité (PEHD) ou tout autre matériau approprié au transport de biogaz.

Un digestat est le produit résidu de la méthanisation et est composé principalement de matière organique non biodégradable, d'eau et de minéraux. Le digestat peut cependant comprendre une partie de matière organique biodégradable, soit en quantité négligeable lorsqu'un taux d'abattement de la matière organique est proche de 100%, soit en quantité plus importante lorsque le taux d'abattement de la matière organique est relativement éloigné de 100%. La sortie du digestat du second espace de réaction peut se faire par tout moyen d'extraction permettant de sortir la matière organique présente au sein dudit espace, tel qu'un moyen par débordement, une vanne ayant au moins une position ouverte permettant le passage de matière organique et une position fermée empêchant le passage de matière organique, un moyen d'aspiration de la matière organique comme une pompe, un moyen de sortie de la matière organique par gravité, un tuyau comprenant un clapet avec une position ouverte et une position fermée. Le second moyen d'extraction peut être tout moyen permettant de sortir un digestat, tel qu'un moyen par débordement, une vanne ayant au moins une position ouverte permettant le passage de digestat et une position fermée empêchant le passage de digestat, un moyen d'aspiration du digestat comme une pompe, un moyen de sortie du digestat par gravité, un tuyau comprenant un clapet avec une position ouverte et une position fermée.

L'étape de sortie de la matière organique du premier espace est activée en fonction d'un degré d'hydrolyse de la partie solide de la matière organique introduite au sein de cet espace. Ce degré d'hydrolyse de la partie solide de la matière organique est défini comme étant un rapport entre d'une part la différence entre la quantité de matière organique présente sous forme soluble après une hydrolyse de la matière organique solide et la quantité de matière organique soluble présente dans la matière organique avant hydrolyse de la matière organique solide (par exemple avant son introduction dans le premier espace de réaction), et d'autre part la quantité de matière organique présente dans la totalité de la matière organique avant une hydrolyse de la matière organique solide (par exemple avant son introduction dans le premier espace de réaction). Ce degré d'hydrolyse peut par exemple être déterminé par la formule suivante :

$$\eta_{hydro} = \frac{DCO_{soluble\ finale} - DCO_{soluble\ initiale}}{DCO_{totale}}$$

Avec DCO exprimée par exemple en milligramme d'oxygène par Litre.

La DCO est la Demande Chimique en Oxygène. Elle peut être déterminée selon la méthode décrite dans la norme ISO 6060:1989 pour la matière organique liquide. Pour mesurer la $DCO_{totale}$, la matière organique solide est préalablement broyée afin d'obtenir des fragments d'une taille maximum de l'ordre du millimètre, pesée et diluée avec de l'eau déminéralisée avant le dosage selon la norme iso 6060 :1989.

La Demande Chimique en Oxygène (DCO) permet d'apprécier la concentration en matières organiques dissoutes, en suspension dans l'eau, ou présente dans la matière organique solide, à travers la quantité d'oxygène nécessaire à leur oxydation chimique totale.

Le degré d'hydrolyse peut par exemple être déterminé à partir de tests chimiques réalisés par un utilisateur ou par un système automatisé. La $DCO_{soluble\ initiale}$ est la DCO de la matière organique soluble avant le début de l'étape d'hydrolyse (par exemple avant son introduction dans le premier espace de réaction). La $DCO_{totale}$ est la somme de la DCO soluble $_{initiale}$ et de la DCO de la matière organique insoluble avant son introduction dans le premier espace de réaction. La DCO soluble $_{finale}$ est la DCO de la matière organique soluble après sa sortie du premier espace de réaction Ainsi, la DCO soluble $_{initiale}$ et la $DCO_{totale}$ de la matière organique introduite dans le premier espace de réaction peuvent être déterminées avant le début de l'étape d'hydrolyse (par exemple avant l'introduction de cette matière organique dans le premier espace de réaction). Des échantillons sont ensuite prélevés par un utilisateur ou contrôlé par un moyen de contrôle présent soit au sein de la sortie de matière organique du premier espace de réaction, soit grâce à un moyen de prélèvement de la matière organique présente dans le premier espace de réaction (tel qu'un robinet par exemple) afin de déterminer la DCO soluble $_{finale}$, jusqu'à l'obtention du degré d'hydrolyse recherché par l'utilisateur. Lorsque le degré d'hydrolyse souhaité est atteint, l'utilisateur ou un système automatisé active le premier moyen d'extraction afin de permettre la sortie de la matière organique présente au sein du premier espace de réaction.

L'étape de sortie est activée en fonction de ce degré d'hydrolyse de la matière organique solide présente au sein du premier espace de réaction. Le degré d'hydrolyse de la matière organique solide détermine donc le moment où le premier moyen d'extraction va être activé. La détermination du degré d'hydrolyse nécessaire pour activer le premier moyen d'extraction est dépendante de la nature de la matière organique solide introduite dans le premier espace. Une matière organique solide lente à se dégrader occupe le premier espace durant une période de temps longue. Ainsi, si une quantité importante de matière organique solide lente à dégrader est à traiter, le degré d'hydrolyse activant l'étape de sortie de la matière organique du premier espace de réaction peut être assez faible, de façon à réduire le temps de séjour de la matière organique dans le premier espace de réaction, tout en hydrolysant une partie suffisante de la matière organique afin d'avoir un rendement global de la réaction de méthanisation satisfaisant. Par exemple, si 60% de la matière organique solide est hydrolysée en 5 jours, mais que le reste de la matière organique solide est hydrolysée en 25 jours, le degré d'hydrolyse nécessaire pour activer le premier moyen d'extraction peut être défini à 60%. En revanche, pour une matière organique solide se dégradant rapidement (comme par exemple des résidus de salade), l'hydrolyse complète de la matière organique est plus rapide (de l'ordre de deux jours), alors, le taux d'hydrolyse nécessaire pour activer le premier moyen d'extraction peut être défini à 80%, plus préférentiellement à 90%, voire encore plus préférentiellement à 100%. Bien entendu, la détermination du degré d'hydrolyse nécessaire pour activer le premier moyen d'extraction peut également être dépendante de la taille du premier espace de réaction en fonction du volume de matière organique à traiter. Si le premier espace de réaction est d'un volume relativement réduit par rapport au volume de matière organique à traiter, le degré d'hydrolyse nécessaire pour activer le premier moyen d'extraction peut être défini à un seuil relativement faible, et inversement, si le premier espace de réaction est d'un volume relativement important par rapport au volume de matière organique à traiter, le degré d'hydrolyse nécessaire pour activer le premier moyen d'extraction peut être défini à un seuil relativement élevé.

Le procédé selon l'invention ne comprend aucune étape de réintroduction de la matière organique solide au sein du premier espace de réaction après que cette matière organique solide ait été extraite du premier espace de réaction. Ainsi, suite à la sortie de la matière organique du premier espace de réaction, la quantité de matière organique disponible pour réaliser les étapes ultérieures de la méthanisation est connue, permettant d'ajuster les paramètres de ces réactions afin d'obtenir un rendement plus élevé. Un temps de séjour de la matière organique dans le second espace de réaction est donc déterminé indépendamment des étapes antérieures du procédé de méthanisation. Ainsi le procédé selon l'invention permet d'avoir un premier espace de réaction de taille plus réduite, plus robuste et dont la quantité de matière organique disponible après sortie du premier espace de réaction pour les étapes ultérieures de la méthanisation est connue et le procédé est maitrisé en fonction des besoins de l'utilisateur, chaque étape du procédé étant indépendante, permettant ainsi l'optimisation et/ou la modulation de chacune de ces étapes.

Selon un mode de réalisation préféré, le procédé selon l'invention comprend les étapes suivantes :

- l'étape de sortie de la matière organique du premier espace de réaction est suivie d'une étape de séparation afin d'obtenir une matière organique liquide séparée et une matière organique solide séparée,
- ladite étape de séparation étant suivie d'une introduction de la matière organique liquide séparée dans le deuxième espace de réaction,
- une acétogenèse et une méthanogenèse de la matière organique liquide séparée est réalisée dans le second espace de réaction,
- la matière organique solide séparée est évacuée par une sortie pour matière organique solide séparée.

Selon ce mode de réalisation préféré de l'invention, une étape de séparation de la matière organique liquide et de la matière organique solide est présente suite à la sortie de matière organique du premier espace de réaction. La séparation peut se faire selon une quelconque méthode connue. Par exemple, la séparation peut se faire par centrifugation, filtration, par une presse à vis d'Archimède, un tamis, par une membrane, par distillation, par évaporation. Il est entendu qu'en fonction du moyen de séparation utilisé, il est possible que la séparation de phase entre la matière organique solide et la matière organique liquide ne soit pas parfaite, résultant en la présence d'une quantité faible de matière organique solide au sein de la matière organique liquide séparée, et inversement, en la présence d'une quantité faible de matière organique liquide au sein de la matière organique solide séparée. La matière organique solide séparée peut être définie comme toute matière organique solide dont la taille ou la densité est supérieure à la limite de séparation du moyen de séparation. Ainsi, si le moyen de séparation est un tamis, la matière organique solide séparée est considérée comme étant toute la matière organique n'ayant pas été en mesure de traverser le tamis.

Après séparation des deux formes de matière organique, la matière organique liquide séparée est introduite dans le deuxième espace de réaction, tandis que la matière organique solide séparée n'est pas introduite dans ce même espace. Ceci permet de traiter la matière organique liquide séparée dans des conditions de réaction optimum, ce que ne permettrait pas la présence de matière organique solide. Ainsi, les conditions de réactions se déroulant au sein du deuxième espace de réaction (acétogenèse et méthanogenèse) sont définies en fonction des propriétés intrinsèques de la matière organique liquide séparée uniquement.

Selon ce mode de réalisation, la matière organique solide séparée n'est pas traitée, et est évacuée par une sortie située après le moyen de séparation. On privilégiera ce procédé lorsque la matière organique introduite dans le premier espace de réaction comprend une matière organique solide rapidement hydrolysable, signifiant que la matière organique solide séparée ne comprend plus qu'une portion relativement négligeable de matière organique solide hydrolysable et donc intéressante dans le cadre d'un procédé de méthanisation, ou que le degré d'hydrolyse déterminant l'activation de la sortie de matière organique du premier espace de réaction est un degré d'hydrolyse élevé (tel que 70%, plus préférentiellement 80% voire encore plus préférentiellement 90%, et encore plus préférentiellement 100%), la matière organique sortie du premier espace de réaction ne présentant alors plus de matière organique solide hydrolysable en quantité suffisante pour que la présence d'un espace de réaction supplémentaire soit utile.

Selon un mode encore plus préféré de réalisation, le procédé selon l'invention est caractérisé en ce que l'étape de sortie du premier digestat du second espace de réaction est activée en fonction d'un taux d'abattement de la matière organique liquide séparée au sein du second espace de réaction.

Par taux d'abattement d'une matière organique liquide séparée, il faut comprendre le rapport entre d'une part la différence entre la quantité de matière organique liquide séparée en entrée du second espace de réaction et la quantité de matière organique en sortie du même second espace de réaction présente dans le digestat, et d'autre part la quantité de matière organique liquide séparée en entrée du second espace de réaction. Ainsi, l'activation de la sortie du digestat ne se fait que lorsque la quantité de matière organique liquide atteint un seuil déterminé. Ce seuil peut correspondre à un seuil ou la matière organique pouvant aboutir à la formation d'un biogaz devient négligeable (du fait par exemple d'une faible concentration), ou peu intéressante en terme de cinétique de réaction.

Le taux d'abattement de la matière organique liquide séparée peut par exemple se calculer avec la formule suivante :

$$Taux\ d'abattement\ DCO_{liquide} = \frac{Quantité\ DCO_{entrant} - Quantité\ DCO_{digestat}}{Quantité\ DCO_{entrant}}$$

La quantité DCO est exprimée par exemple en milligramme d'oxygène.

La quantité de DCO $_{entrant}$ peut être déterminée par son dosage au sein de la matière organique liquide séparée avant son entrée dans le second espace de réaction par la méthode décrite dans la norme ISO 6060 :1989. La quantité de DCO $_{digestat}$ peut être déterminée par son dosage au sein d'un échantillon de digestat sorti du second espace de réaction ou par son dosage au sein du second espace de réaction par la méthode décrite dans la norme ISO 6060 :1989.

Selon un mode de réalisation préférentiel, le procédé de méthanisation selon l'invention est caractérisé en ce que :

- l'étape d'évacuation de la matière organique solide séparée est suivie de l'introduction de ladite matière organique solide séparée dans un troisième espace de réaction,
- une hydrolyse, une acidogenèse, une acétogenèse et une méthanogenèse de la matière organique solide séparée est réalisée dans le troisième espace de réaction,
- un second digestat est sorti du troisième espace de réaction par un troisième moyen d'extraction.

Selon ce mode de réalisation préféré de l'invention, l'étape de séparation de la matière organique liquide et de la matière organique solide est suivie de l'introduction de la matière organique solide séparée au sein d'un troisième espace de réaction. Cette étape d'introduction peut se faire au moyen d'une vanne, d'un débordement, d'une pompe ou de tout autre moyen de transfert reliant la sortie pour matière organique solide séparée du moyen de séparation au troisième espace de réaction.

La matière organique solide séparée est insérée dans le troisième espace de réaction, tandis que la matière organique liquide séparée n'est pas introduite dans ce même espace. Ceci permet de traiter la matière organique solide séparée dans des conditions de réaction optimum, ce que ne permettrait pas la présence de matière organique liquide. Ainsi, les conditions de réactions se déroulant au sein du troisième espace de réaction sont définies en fonction des propriétés intrinsèques de la matière organique solide séparée uniquement. Il faut noter que l'ensemble des étapes de la méthanisation peuvent se dérouler au sein de ce troisième espace de réaction. Ainsi, les étapes d'hydrolyse, d'acidogenèse, d'acétogenèse et de méthanogenèse peuvent se dérouler dans ce troisième espace de réaction.

Selon ce mode de réalisation, la matière organique solide séparée est également traitée. On privilégiera ce procédé lorsque la matière organique introduite dans le premier espace de réaction comprend une matière organique solide lentement hydrolysable et/ou que le degré d'hydrolyse déterminé pour activer la sortie de matière organique du premier espace de réaction est relativement faible, signifiant que la matière organique solide séparée comprend une portion substantielle de matière organique solide hydrolysable et donc intéressante dans le cadre d'un procédé de méthanisation en vue de la production d'un biogaz.

Selon ce mode de réalisation, de façon préférentielle, l'étape de sortie du second digestat du troisième espace de réaction est activée en fonction d'un taux d'abattement de la matière organique solide séparée au sein du troisième espace de réaction.

Par taux d'abattement de la matière organique solide séparée, il faut comprendre le rapport entre d'une part la différence entre la quantité de matière organique solide séparée en entrée du troisième espace de réaction et la quantité de matière organique en sortie du même troisième espace de réaction présent dans le digestat, et d'autre part la quantité de matière organique solide séparée en entrée du troisième espace de réaction. Ainsi, l'activation de la sortie du digestat ne se fait que lorsque la quantité de matière organique solide atteint un seuil déterminé. Ce seuil peut correspondre à un seuil ou la matière organique pouvant aboutir à la formation d'un biogaz devient négligeable (du fait par exemple d'une faible concentration), ou peu intéressante en terme de cinétique de réaction.

Le taux d'abattement de la matière organique solide séparée peut par exemple se calculer selon la formule suivante :

$$Taux\ d'abattement\ DCO_{solide} = \frac{Quantité\ DCO_{entrant} - Quantité\ DCO_{digestat}}{Quantité\ DCO_{entrant}}$$

Avec quantité DCO exprimée en milligramme d'oxygène.

La quantité DCO $_{entrant}$ peut être déterminée par son dosage au sein de la matière organique solide séparée avant son entrée dans le troisième espace de réaction par la méthode décrite dans la norme ISO 6060:1989, la matière organique solide étant préalablement broyée, pesée, et diluée. La quantité de DCO $_{digestat}$ peut être déterminée par son dosage au sein d'un échantillon de digestat sorti du troisième espace de réaction, ou par son dosage au sein du troisième espace de réaction, le dosage étant fait sur le digestat liquide, et solide s'il est présent, par la méthode décrite dans la norme ISO 6060:1989 pour le digestat liquide et pour le digestat solide s'il est présent, le digestat solide étant préalablement broyé, pesé, et dilué avant le dosage.

De façon toujours préférentielle, le procédé selon ce mode de réalisation peut être réalisé de façon à ce que l'étape de sortie du premier digestat du second espace de réaction et l'étape de sortie du second digestat du troisième espace de réaction soient activées indépendamment l'une de l'autre.

Selon ce mode de réalisation, les moyens de sortie du digestat d'un second et d'un troisième espace de réaction sont activés indépendamment l'un de l'autre, de façon préférentielle en fonction de chacun des taux d'abattement de la matière organique présente au sein de chacun des espaces de réaction, afin d'avoir un procédé plus efficient au sein de chacun des espaces de réaction en ayant des temps de séjour de la matière organique liquide et de la matière organique solide indépendants l'un de l'autre, et définis en fonction des qualités intrinsèques de chacune des formes de matière organique, et de l'efficacité ou du rendement désiré par l'utilisateur.

Selon un mode de réalisation préféré, l'un quelconque des procédés déjà décrits peut comprendre une étape où au moins une partie de la matière organique liquide séparée présente au sein du second espace de réaction est introduite dans le premier espace de réaction.

Dans ce mode de réalisation, l'étape d'introduction de matière organique liquide séparée présente dans le second espace de réaction dans le premier espace de réaction peut être réalisée grâce à une vanne suivie d'un tuyau reliant les deux espaces de réaction. Cette introduction peut être contrôlée par un moyen de contrôle permettant l'introduction ou non de matière organique liquide. Il s'entend que le volume de la matière organique liquide ainsi transférée peut être déterminé, et que la DCO de cette matière organique liquide transférée peut être déterminée afin d'en tenir compte lors de la détermination d'un taux d'abattement de la matière organique liquide séparée au sein du second espace de réaction et pour en tenir compte lors de la détermination du degré d'hydrolyse au sein du premier espace de réaction, afin de conserver des conditions optimales de réaction au sein de chacun des espaces de réaction. La détermination de la DCO présente au sein d'un flux de matière organique peut être déterminée selon la norme ISO 6060:1989. Cette étape peut être mise en oeuvre lorsque la matière organique présente dans le premier espace de réaction présente des concentrations trop importantes en acides gras volatils, ou lorsque la matière organique au sein de ce premier espace de réaction est essentiellement constituée de matière solide et/ou que le contenu de ce premier espace de réaction est difficilement agitable et/ou homogénéisable en l'état.

Selon ce mode de réalisation préféré, il est possible que l'introduction de ladite matière organique liquide séparée dans le premier espace de réaction soit contrôlée afin de maintenir au sein du premier espace de réaction une concentration en acides gras volatils inférieure à un seuil. Ce seuil peut par exemple être choisi comme étant un seuil au-delà duquel la réaction d'acidogenèse est inhibée. Cette inhibition peut être déterminée par des dosages chimiques connus de l'homme du métier.

Maintenir la concentration en acides gras volatils inférieure à un seuil déterminé permet de maintenir une cinétique d'acidogenèse satisfaisante dans le cadre d'un procédé de méthanisation.

Selon un mode de réalisation préférentiel ou alternatif, il est possible que le procédé de méthanisation comprenne une étape où au moins une partie de la matière organique liquide séparée est introduite dans le premier espace de réaction avant son introduction dans le second espace de réaction.

Cette étape peut se réaliser par exemple à l'aide d'une vanne suivie d'un tuyau reliant une sortie pour matière organique liquide séparée du moyen de séparation au premier espace de réaction.

Selon ce mode préférentiel ou alternatif, l'introduction de la matière organique liquide séparée dans le premier espace de réaction avant son introduction dans le second espace de réaction est contrôlée afin de maintenir un pH par exemple inférieur à 5,5 au sein du premier espace de réaction.

Cette introduction de matière organique liquide séparée vers le premier espace de réaction peut être contrôlée par tout moyen, comme par exemple un moyen de régulation programmé avec une consigne, cette consigne étant modulable en fonction du pH au sein du premier espace de réaction, la consigne permettant ou non le passage d'un flux plus ou moins important de matière organique liquide en fonction du pH au sein du premier espace de réaction. Le contrôle du pH au sein du premier espace de réaction peut se faire par n'importe quel moyen connu, tel que l'utilisation d'un pH-mètre situé au sein du premier espace de réaction. Il est également possible de contrôler le pH de la matière organique liquide séparée concomitamment, afin de déterminer le volume de matière organique liquide séparée à transférer en fonction du pH de la matière organique liquide séparée et du pH de la matière organique présente au sein du premier espace de réaction, du volume du premier espace de réaction et/ou de la quantité de matière organique présente dans le premier espace de réaction, et ainsi modifier la consigne du moyen de régulation. Il s'entend que le volume de la matière organique liquide ainsi transférée peut être déterminé, et que la DCO de cette matière organique liquide transférée peut être déterminée afin d'en tenir compte lors de la détermination du degré d'hydrolyse au sein du premier espace de réaction, afin de conserver des conditions optimales de réaction de ce premier espace.

Dans un mode de réalisation préféré, le procédé de méthanisation comprend en outre une étape où au moins une partie de la matière organique liquide séparée présente au sein du second espace de réaction est introduite dans le troisième espace de réaction.

Cette étape peut être mise en oeuvre lorsque la matière organique solide séparée présente dans le troisième espace de réaction ne présente pas des qualités suffisantes de viscosité afin d'être agitable au sein du troisième espace de réaction, par exemple lorsque la matière organique au sein de ce troisième espace de réaction est essentiellement constituée de matière organique solide de très grande taille ou particulièrement solide (comme par exemple des résidus ligneux). Ainsi, il est possible d'agiter le contenu du troisième espace de réaction en cas de besoin. L'introduction de matière organique liquide séparée du second espace de réaction vers le troisième espace de réaction peut se faire grâce à une vanne suivie d'un tuyau reliant ces deux espaces de réaction, ou tout moyen équivalent. Cette vanne peut, au besoin, être contrôlée par un moyen de contrôle ou de régulation, et l'introduction de matière organique liquide séparée peut dépendre de différents paramètres, comme par exemple la viscosité du contenu du troisième espace de réaction, le taux de matière sèche du contenu du troisième espace de réaction. Le moyen de contrôle ou de régulation peut comprendre une consigne, cette consigne étant modulable en fonction de ces paramètres. Il s'entend que le volume de la matière organique liquide ainsi transférée peut être déterminé, et que la DCO de cette matière organique liquide transférée peut être déterminée afin d'en tenir compte lors de la détermination des taux d'abattement au sein de chacun des espaces de réaction.

L'invention concerne également un appareil de méthanisation (50) afin de mettre en oeuvre au moins un procédé selon l'invention tel que décrit précédemment.

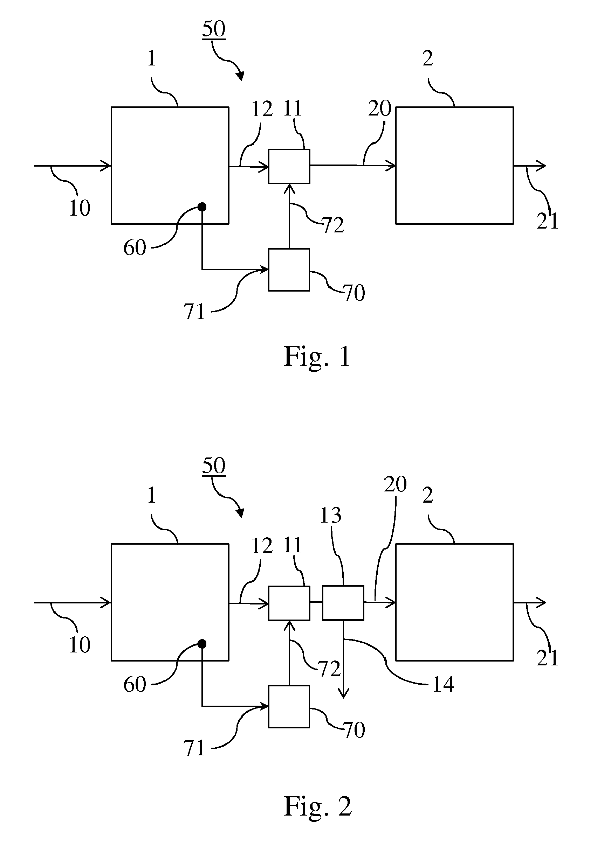

Tel qu'illustré à la fig. 1, l'invention concerne également un appareil de méthanisation (50) de matière organique au moins en partie solide, ledit appareil comprenant :

- un premier espace de réaction (1) conçu pour y effectuer une hydrolyse ainsi qu'une acidogenèse de la matière organique et comprenant une première entrée de matière organique (10) ainsi qu'une première sortie de matière organique (12),
- un second espace de réaction (2) conçu pour y effectuer une acétogenèse ainsi qu'une méthanogenèse de matière organique et comprenant une deuxième entrée de matière organique (20) ainsi qu'une deuxième sortie d'un digestat (21),
- des premiers moyens d'extraction commandables (11) conçus pour extraire de la matière organique du premier espace via la première sortie (12) et pour transférer cette matière organique vers la deuxième entrée (20),

caractérisé en ce que ledit appareil comprend en outre :

- des premiers moyens de mesure (60) d'un degré d'hydrolyse de la partie solide de la matière organique solide contenue dans le premier espace (1),
- un premier contrôleur (70) ayant une première entrée de mesure (71) reliée aux premiers moyens de mesure et une première sortie de commande (72) reliée aux premiers moyens d'extraction, ledit premier contrôleur (70) étant configuré pour commander une activation des premiers moyens d'extraction en fonction d'un degré d'hydrolyse fourni par les premiers moyens de mesure (60),

et en ce qu'aucun moyen de retour de matière organique solide provenant du premier espace de réaction (1) vers ledit premier espace de réaction (1) n'est présent.

Le premier espace de réaction (1) peut être n'importe quel espace permettant à une matière organique au moins en partie solide de subir au moins en partie une hydrolyse et une acidogenèse. Ce premier espace de réaction peut par exemple être une cuve d'hydrolyse.

Une première entrée pour matière organique (10) peut être n'importe quel moyen permettant d'introduire une matière organique au moins en partie solide dans le premier espace de réaction (1), comme par exemple une ouverture, une trappe, un système de vis d'Archimède, une vanne. Une première sortie pour matière organique (12) peut être n'importe quel moyen permettant de sortir la matière organique sous forme solide et liquide du premier espace de réaction (1), comme par exemple une vanne poursuivie d'un tuyau, une trappe.

Un second espace de réaction (2) peut être n'importe quel espace permettant à une matière organique de subir au moins en partie une réaction d'acétogenèse et une réaction de méthanogenèse dans le but de produire un biogaz, et notamment du méthane. Le second espace de réaction (2) peut également comprendre une sortie dédiée au biogaz produit, se présentant par exemple sous la forme d'une canalisation en inox ou en polyéthylène haute densité (PEHD).

Une seconde entrée pour matière organique (20) peut être n'importe quel moyen permettant d'introduire une matière organique dans le second espace de réaction (2), comme par exemple une ouverture, une trappe, un système de vis d'Archimède, un tuyau, une vanne. Une première sortie d'un digestat (21) peut être n'importe quel moyen permettant de sortir le digestat du second espace de réaction (2), comme par exemple une vanne poursuivie d'un tuyau, une trappe.

Des premiers moyens d'extraction commandables (11) peuvent être une vis sans fin motorisée, une pompe, une électrovanne.

Des premiers moyens de mesure (60) d'un degré d'hydrolyse de la matière organique solide contenue dans le premier espace peuvent être un dispositif permettant de réaliser les dosages de la matière organique présente dans la matière organique liquide et dans la matière organique solide présentes dans le premier espace de réaction (1). Avantageusement, ces premiers moyens de mesure peuvent être un DCO mètre.

Un premier contrôleur (70) peut par exemple être un microprocesseur ou un ordinateur, dont une première entrée de mesure (71) est reliée aux premiers moyens de mesure (60) par exemple grâce à un câble de communication connecté par une de ses extrémités aux premiers moyens de mesure (60) et par une autre extrémité à un port entrant du premier contrôleur (70). Le premier contrôleur (70) possède également une première sortie de commande (72) par exemple un port sortant, et un câble de communication relie ce port sortant aux premiers moyens d'extraction commandables (11). De façon préférentielle, le premier contrôleur (70) est configuré pour activer les premiers moyens d'extraction commandables (11) lorsque le degré d'hydrolyse de la matière organique solide présente dans le premier espace de réaction (1) atteint un seuil d'hydrolyse de la matière organique solide défini.

Selon un mode de réalisation préféré illustré à la figure 2, l'appareil de méthanisation (50) comprend en outre un moyen de séparation (13) apte à séparer une matière organique entrante en une matière organique liquide séparée et une matière organique solide séparée, ledit moyen de séparation (13) étant intercalé entre les premiers moyens d'extraction (11) et la deuxième entrée (20), ladite matière organique entrante provenant des premiers moyens d'extraction commandables (11), ladite matière organique liquide séparée alimentant la deuxième entrée (20), et ladite matière organique solide séparée étant dirigée vers une sortie pour matière organique solide séparée (14).

Un moyen de séparation (13) peut être n'importe quel moyen permettant de séparer une matière organique liquide et une matière organique solide mélangées afin d'obtenir séparément une matière organique liquide séparée et une matière organique solide séparée. Un moyen de séparation (13) peut par exemple être une centrifugeuse, une presse à vis, un séparateur à débordement, un tamis, un filtre, un moyen de séparation par décantation ou par sédimentation.

La sortie pour matière organique solide séparée (14) peut être n'importe quel moyen permettant de sortir la matière organique solide du moyen de séparation (13), comme par exemple un tuyau, une trappe.

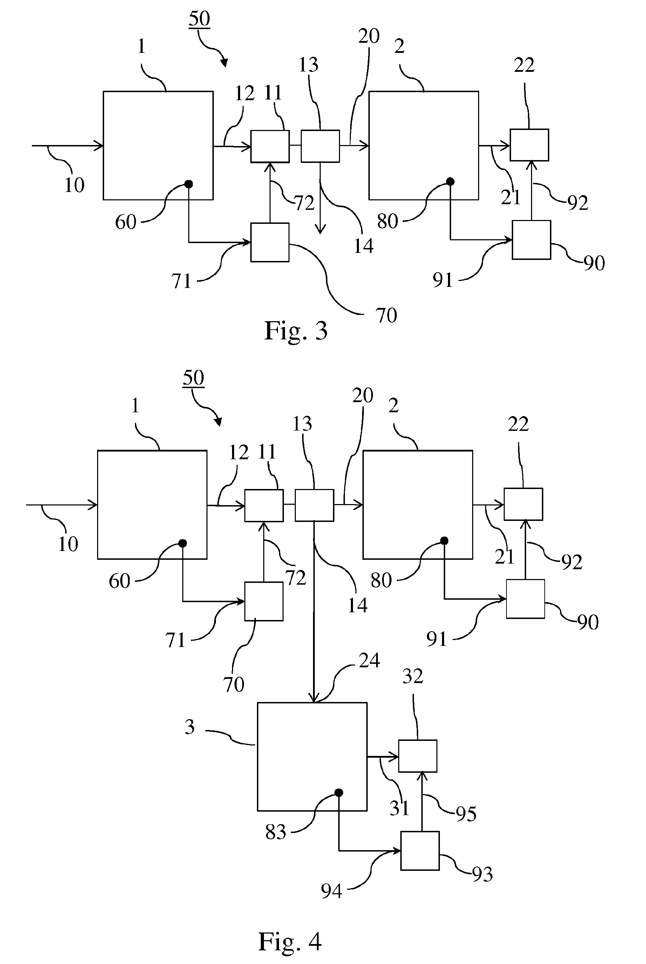

Selon un mode de réalisation encore plus préféré illustré à la figure 3, l'appareil de méthanisation (50) comprend en outre :

- des seconds moyens d'extraction commandables (22) conçus pour extraire le digestat du second espace via la deuxième sortie (21),

- des seconds moyens de mesure (80) d'un taux d'abattement de la matière organique liquide séparée contenue dans le second espace (2),
- un second contrôleur (90) ayant une seconde entrée de mesure (91) reliée aux seconds moyens de mesure (80) et une seconde sortie de commande (92) reliée aux seconds moyens d'extraction (22), ledit second contrôleur (90) étant configuré pour commander une activation des seconds moyens d'extraction (22) en fonction d'un taux d'abattement fourni par les seconds moyens de mesure (80).

Les seconds moyens d'extraction commandables (22) peuvent être une vis sans fin motorisée, une pompe, une électrovanne. Les seconds moyens de mesure (80) d'un taux d'abattement de la matière organique liquide séparée peuvent être un dispositif permettant de réaliser les dosages de la matière organique présente dans le second espace de réaction (2). Avantageusement, ces seconds moyens de mesure peuvent être un DCO mètre couplé à un dispositif de mesure de la masse et/ou du volume de la matière organique introduite dans le second espace de réaction (2).

Un second contrôleur (90) peut par exemple être un microprocesseur ou un ordinateur, dont une seconde entrée de mesure (91) est reliée aux seconds moyens de mesure (80) par exemple grâce à un câble de communication connecté par une de ses extrémités aux seconds moyens de mesure (80) et par une autre extrémité à un port entrant du second contrôleur (90). Le second contrôleur (90) possède également une seconde sortie de commande (92) par exemple un port sortant, et un câble de communication relie ce port sortant aux seconds moyens d'extraction commandables (22). De façon préférentielle, le second contrôleur (90) est configuré pour activer les seconds moyens d'extraction commandables (22) lorsque le taux d'abattement de la matière organique liquide séparée présente dans le second espace de réaction (2) atteint un taux d'abattement de la matière organique liquide séparée défini.

Selon un mode de réalisation encore plus préféré illustré à la figure 4, l'appareil de méthanisation (50) comprend en outre :

- un troisième espace de réaction (3) conçu pour y effectuer une hydrolyse, une acidogenèse, une acétogenèse ainsi qu'une méthanogenèse de matière organique solide séparée et comprenant une troisième entrée de matière organique (24) ainsi qu'une seconde sortie d'un digestat (31), ladite troisième entrée de matière organique (24) étant connecté à la sortie pour matière organique solide séparée (14), ladite matière organique solide séparée provenant de ladite sortie pour matière organique solide séparée (14), ladite matière organique solide séparée alimentant le troisième espace de réaction (3),
- des troisièmes moyens d'extraction commandables (32) conçus pour extraire le digestat du troisième espace (3) via la seconde sortie d'un digestat (31),
- des troisièmes moyens de mesure (83) d'un taux d'abattement de la matière organique solide séparée contenue dans le troisième espace (3),
- un troisième contrôleur (93) ayant une troisième entrée de mesure (94) reliée aux troisièmes moyens de mesure (83) et une troisième sortie de commande (95) reliée aux troisièmes moyens d'extraction commandables (32), ledit troisième contrôleur (93) étant configuré pour commander une activation des troisièmes moyens d'extraction (32) en fonction d'un taux d'abattement fourni par les troisièmes moyens de mesure (83).

La troisième espace de réaction (3) peut être n'importe quel espace permettant à une matière organique solide de subir au moins en partie des réactions d'hydrolyse, d'acidogenèse, d'acétogenèse et de méthanogenèse, tel que par exemple un digesteur infiniment mélangé (CSTR), un digesteur voie sèche (type garage ou système grange). Le troisième espace de réaction (3) peut également comprendre une sortie dédiée au biogaz produit, se présentant par exemple sous la forme d'une canalisation en inox ou en PEHD. Cette sortie pour biogaz peut être connectée à la sortie pour biogaz présente sur le second espace de réaction (2). La seconde sortie d'un digestat (31) peut être n'importe quel moyen permettant de sortir le digestat du troisième espace de réaction (3), comme par exemple une vanne poursuivie d'un tuyau, une trappe. La troisième entrée de matière organique (24) peut être n'importe quel moyen permettant d'introduire la matière organique solide séparée provenant du moyen de séparation (13) via la sortie pour matière organique séparée (14) comme par exemple une ouverture, une trappe, un tuyau.

Les troisièmes moyens d'extraction commandables (32) peuvent être une vis sans fin motorisée, une pompe, une électrovanne. Les troisièmes moyens de mesure (83) d'un taux d'abattement de la matière organique solide séparée peuvent être un dispositif permettant de réaliser les dosages de la matière organique présente dans le troisième espace de réaction (3). Avantageusement, ces troisièmes moyens de mesure peuvent être un DCO mètre couplé à un dispositif de mesure de la masse de la matière organique introduite dans le troisième espace de réaction (3).

Un troisième contrôleur (93) peut par exemple être un microprocesseur ou un ordinateur, dont une troisième entrée de mesure (94) est reliée aux troisièmes moyens de mesure (83) par exemple grâce à un câble de communication connecté par une de ses extrémités aux troisièmes moyens de mesure (83) et par une autre extrémité à un port entrant du troisième contrôleur (93). Le troisième contrôleur (93) possède également une troisième sortie de commande (95) par exemple un port sortant, et un câble de communication relie ce port sortant aux troisièmes moyens d'extraction commandables (32). De façon préférentielle, le troisième contrôleur (93) est configuré pour activer les troisièmes moyens d'extraction commandables (32) lorsque le taux d'abattement de la matière organique solide séparée présente dans le troisième espace de réaction (3) atteint un taux d'abattement de la matière organique solide séparée défini.

Selon l'un quelconque des modes de réalisation d'un appareil de méthanisation décrit, le second espace de réaction (2) est un réacteur acceptant une charge égale ou supérieure à 5 kg MV/m$^3$.j.

Un réacteur acceptant une charge égale ou supérieure à 5 kg MV/m$^3$.j peut par exemple être un méthaniseur à lit fixe décrit dans le demande de brevet EP2207873, ou tout autre méthaniseur pour liquide répondant à ce critère (comme par exemple un méthaniseur « Upflow Anaerobic Sludge Blanket » (UASB) ou un méthaniseur « Expended Granule Sludge Blanket » (EGSB)). Par charge, il faut comprendre la quantité de Matière volatile (MV) introduite par unité de volume du second espace de réaction et par jour. La matière volatile est définie comme la matière biodégradable présente au sein du second espace de réaction. Par exemple,

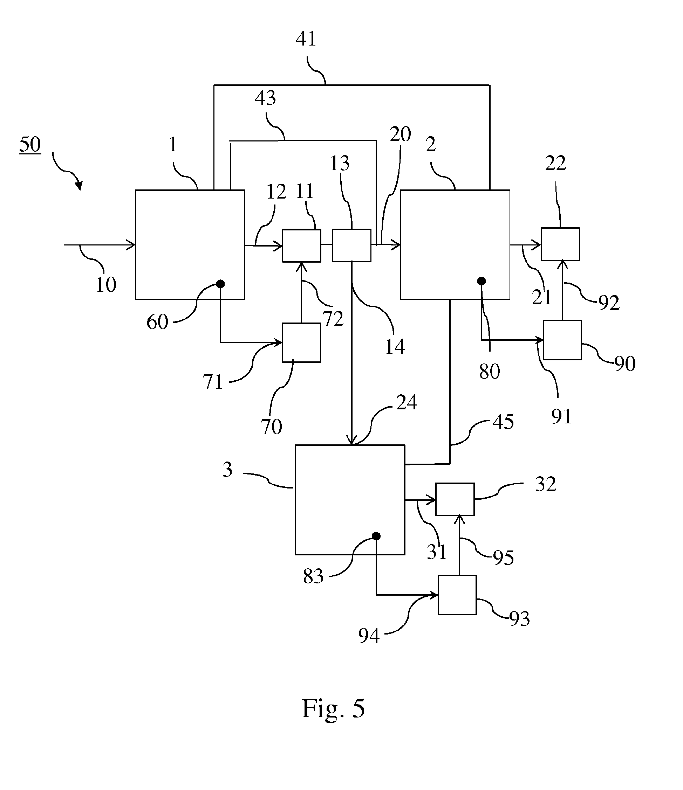

Selon un mode de réalisation préféré illustré à la figure 5, l'appareil de méthanisation (50) comprend un premier moyen d'introduction (41) de la matière organique liquide séparée présente au sein du second espace de réaction (2) dans le premier espace de réaction (1).

Le premier moyen d'introduction (41) peut être n'importe quel moyen permettant de relier le second espace de réaction (2) au premier espace de réaction (1), tout en permettant à un flux de matière organique liquide d'être dirigé du second espace de réaction (2) vers le premier espace de réaction (1). Ce premier moyen d'introduction (41) peut par exemple être une vanne suivie d'un tuyau. Ce premier moyen d'introduction (41) peut comprendre des moyens de pompage commandables conçus pour extraire la matière organique liquide du second espace de réaction (2) et introduire la matière organique liquide dans le premier espace de réaction (1).

Selon un mode de réalisation préféré et illustré à la figure 5, l'appareil de méthanisation (50) comprend un second moyen d'introduction (43) de la matière organique liquide séparée avant son introduction dans le second espace de réaction (2) dans le premier espace de réaction (1).

Le second moyen d'introduction (43) peut être n'importe quel moyen permettant d'introduire de la matière organique liquide séparée, mais non introduite dans le second espace de réaction (2), dans le premier espace de réaction (1), comme par exemple un tuyau relié par l'une de ses extrémités à l'appareil de méthanisation (50) entre le moyen de séparation (13) et la deuxième entrée (20), et par une autre extrémité au premier espace de réaction (1). Ce second moyen d'introduction (43) peut comprendre des moyens de pompage commandables conçus pour extraire la matière organique liquide après séparation et l'introduire dans le premier espace de réaction (1).

Selon un mode de réalisation encore plus préféré illustré à la figure 5, l'appareil de méthanisation (50) comprend un troisième moyen d'introduction (45) de la matière organique liquide séparée présente au sein du second espace de réaction (2) dans le troisième espace de réaction (3).

Le troisième moyen d'introduction (45) peut être n'importe quel moyen permettant de relier le second espace de réaction (2) et le troisième espace de réaction (3), tout en permettant à un flux de matière organique liquide d'être dirigé du second espace de réaction (2) vers le troisième espace de réaction (3). Ce moyen peut par exemple être une vanne suivie d'un tuyau comprenant ou non une pompe. Ce troisième moyen d'introduction (45) peut comprendre des moyens de pompage commandables conçus pour extraire la matière organique liquide séparée présente au sein du second espace de réaction (2) et l'introduire dans le troisième espace de réaction (3).

Selon un mode de réalisation préféré illustré à la figure 5, l'appareil de méthanisation (50) comprend un premier moyen d'introduction (41) de la matière organique liquide séparée présente au sein du second espace de réaction (2) dans le premier espace de réaction (1), un second moyen d'introduction (43) de la matière organique liquide séparée dans le premier espace de réaction et un troisième moyen d'introduction (45) de la matière organique liquide séparée présente au sein du second espace de réaction (2) dans le troisième espace de réaction (3).

La présente invention a été décrite en relation avec des modes de réalisations spécifiques, qui ont une valeur purement illustrative et ne doivent pas être considérés comme limitatifs. D'une manière générale, il apparaîtra évident pour l'homme du métier que la présente invention n'est pas limités aux exemples illustrés et/ou décrits ci-dessus. La présence de numéros de référence aux dessins ne peut être considérée comme limitative, y compris lorsque ces numéros sont indiqués dans les revendications.

L'usage des verbes « comprendre », « inclure », « comporter », ou toute autre variante, ainsi que leurs conjugaisons, ne peut en aucune façon exclure la présence d'éléments autres que ceux mentionnés. L'usage de l'article indéfini « un », « une », ou de l'article défini « le », « la » ou « l' », pour introduire un élément n'exclut pas la présence d'une pluralité de ces éléments.

L'invention peut également être décrite comme suit : l'invention concerne un procédé de méthanisation de matière organique en deux étapes dans lequel les paramètres de chacune des étapes sont contrôlés indépendamment les uns des autres afin d'obtenir un rendement global de réaction satisfaisant en fonction de la nature de la matière organique utilisée lors du procédé de méthanisation. L'invention concerne également un appareil pour mettre en oeuvre le procédé selon l'invention.

**Revendications**

1. Procédé de méthanisation d'une matière organique au moins en partie solide comprenant les étapes de :

   - introduction de ladite matière organique dans un premier espace de réaction,
   - hydrolyse d'au moins une partie de la matière organique solide et acidogenèse de la matière organique au sein dudit premier espace de réaction,
   - sortie de la matière organique du premier espace de réaction par un premier moyen d'extraction,
   - introduction d'au moins une partie de la matière organique sortie du premier espace de réaction dans un second espace de réaction,
   - acétogenèse et méthanogenèse de la matière organique présente au sein du second espace de réaction,
   - sortie d'un premier digestat du second espace de réaction par un second moyen d'extraction,

   **caractérisé en ce que** l'étape de sortie de la matière organique du premier espace de réaction est activée en fonction d'un degré d'hydrolyse de la partie solide de la matière organique introduite au sein dudit premier espace de réaction,
   et **en ce qu'**après la sortie de matière organique du premier espace de réaction, aucune partie solide de la matière organique extraite n'est réintroduite dans ledit premier espace de réaction.

2. Procédé de méthanisation selon la revendication 1 **caractérisé en ce que**

   - l'étape de sortie de la matière organique du premier espace de réaction est suivie d'une étape de séparation afin d'obtenir une matière organique liquide séparée et une matière organique solide séparée,
   - ladite étape de séparation étant suivie d'une introduction de la matière organique liquide séparée dans le deuxième espace de réaction,
   - une acétogenèse et une méthanogenèse de la matière organique liquide séparée est réalisée dans le second espace de réaction,
   - la matière organique solide séparée est évacuée par une sortie pour matière organique solide séparée.

3. Procédé de méthanisation selon la revendication 2, **caractérisé en ce que** l'étape de sortie du premier digestat du second espace de réaction est activée en fonction d'un taux d'abattement de la matière organique liquide séparée au sein du second espace de réaction.

4. Procédé de méthanisation selon l'une quelconque des revendications 2 et 3 et **caractérisé en ce que**

   - l'étape d'évacuation de la matière organique solide séparée est suivie de l'introduction de ladite matière organique solide séparée dans un troisième espace de réaction,
   - une hydrolyse, une acidogenèse, une acétogenèse et une méthanogenèse de la matière organique solide séparée est réalisée dans le troisième espace de réaction,
   - un second digestat est sorti du troisième espace de réaction par un troisième moyen d'extraction.

5. Procédé de méthanisation selon la revendication 4, **caractérisé en ce que** l'étape de sortie du second digestat du troisième espace de réaction est activée en fonction d'un taux d'abattement de la matière organique solide séparée au sein du troisième espace de réaction.

6. Procédé selon la revendication 5, **caractérisé en ce que** l'étape de sortie du premier digestat du second espace de réaction et l'étape de sortie du second digestat du troisième espace de réaction sont activées indépendamment l'une de l'autre.

7. Procédé de méthanisation selon l'une quelconque des revendications précédentes, **caractérisé en ce qu'**au moins une partie de la matière organique liquide présente au sein du second espace de réaction est introduite dans le premier espace de réaction.

8. Procédé de méthanisation selon la revendication 7, **caractérisé en ce que** l'introduction de ladite matière organique liquide séparée dans le premier espace de réaction est contrôlée afin de maintenir au sein du premier espace de réaction une concentration en acides gras volatils inférieure à un seuil d'inhibition de l'acidogenèse.

9. Procédé de méthanisation selon l'une quelconque des revendications 2 à 7, **caractérisé en ce qu'**au moins une

13

partie de la matière organique liquide séparée est introduite dans le premier espace de réaction avant son introduction dans le second espace de réaction.

10. Procédé selon la revendication 9, **caractérisé en ce que** l'introduction de la matière organique liquide séparée dans le premier espace de réaction avant son introduction dans le second espace de réaction est contrôlée afin de maintenir un pH inférieur à 5,5 au sein du premier espace de réaction.

11. Procédé de méthanisation selon l'une quelconque des revendications 4 à 10, **caractérisé en ce qu'**au moins une partie de la matière organique liquide séparée présente au sein du second espace de réaction est introduite dans le troisième espace de réaction.

12. Appareil de méthanisation (50) de matière organique au moins en partie solide, ledit appareil comprenant :

- un premier espace de réaction (1) conçu pour y effectuer une hydrolyse ainsi qu'une acidogenèse de la matière organique et comprenant une première entrée de matière organique (10) ainsi qu'une première sortie de matière organique (12),
- un second espace de réaction (2) conçu pour y effectuer une acétogenèse ainsi qu'une méthanogenèse de matière organique et comprenant une deuxième entrée de matière organique (20) ainsi qu'une première sortie d'un digestat (21),
- des premiers moyens d'extraction commandables (11) conçus pour extraire de la matière organique du premier espace via la première sortie (12) et pour transférer cette matière organique vers la deuxième entrée (20),

**caractérisé en ce que** ledit appareil comprend en outre

- des premiers moyens de mesure (60) d'un degré d'hydrolyse de la partie solide de la matière organique solide contenue dans le premier espace (1),
- un premier contrôleur (70) ayant une première entrée de mesure (71) reliée aux premiers moyens de mesure et une première sortie de commande (72) reliée aux premiers moyens d'extraction commandables (11), ledit premier contrôleur étant configuré pour commander une activation des premiers moyens d'extraction commandables (11) en fonction d'un degré d'hydrolyse fourni par les premiers moyens de mesure (60),

et **en ce qu'**aucun moyen de retour de matière organique solide provenant du premier espace de réaction (1) vers ledit premier espace de réaction (1) n'est présent.

13. Appareil de méthanisation selon la revendication 12, comprenant en outre un moyen de séparation (13) apte à séparer une matière organique entrante en une matière organique liquide séparée et une matière organique solide séparée, ledit moyen de séparation (13) étant intercalé entre les premiers moyens d'extraction (11) et la deuxième entrée (20), ladite matière organique entrante provenant des premiers moyens d'extraction (11), ladite matière organique liquide séparée alimentant la deuxième entrée (20), et ladite matière organique solide séparée étant dirigée vers une sortie pour matière organique solide séparée (14).

14. Appareil de méthanisation selon la revendication 13, comprenant en outre :

- des seconds moyens d'extraction commandables (22) conçus pour extraire le digestat du second espace via la deuxième sortie (21),
- des seconds moyens de mesure (80) d'un taux d'abattement de la matière organique liquide séparée contenue dans le second espace (2),
- un second contrôleur (90) ayant une seconde entrée de mesure (91) reliée aux seconds moyens de mesure et une seconde sortie de commande (92) reliée aux seconds moyens d'extraction (22), ledit second contrôleur (90) étant configuré pour commander une activation des seconds moyens d'extraction (22) en fonction d'un taux d'abattement fourni par les seconds moyens de mesure (80).

15. Appareil de méthanisation selon la revendication 13 ou 14, comprenant en outre :

- un troisième espace de réaction (3) conçu pour y effectuer une hydrolyse, une acidogenèse, une acétogenèse ainsi qu'une méthanogenèse de matière organique solide séparée et comprenant une troisième entrée de matière organique (24) ainsi qu'une seconde sortie d'un digestat (31), ladite troisième entrée de matière organique (24) étant connecté à la sortie pour matière organique solide séparée (14), ladite matière organique solide séparée provenant de ladite sortie pour matière organique solide séparée (14), ladite matière organique solide séparée alimentant le troisième espace de réaction (3),
- des troisièmes moyens d'extraction commandables (32) conçus pour extraire le digestat du troisième espace (3) via la seconde sortie d'un digestat (31),
- des troisièmes moyens de mesure (83) d'un taux d'abattement de la matière organique solide séparée contenue dans le troisième espace (3), - un troisième contrôleur (93) ayant une troisième entrée de mesure (94) reliée aux troisièmes moyens de mesure (83) et une troisième sortie de commande (95) reliée aux troisièmes moyens d'extraction commandables (32), ledit troisième contrôleur (83) étant configuré pour commander une activation des troisièmes moyens d'extraction (32) en fonction d'un taux d'abattement fourni par les troisièmes moyens de mesure (83).

Fig. 1

Fig. 2

Fig. 3

Fig. 4

Fig. 5

| | Europäisches Patentamt European Patent Office Office européen des brevets | **RAPPORT DE RECHERCHE EUROPEENNE** | Numéro de la demande EP 13 16 6282 |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2012/125840 A1 (SMITH CHARLES SATISH [US]) 24 mai 2012 (2012-05-24) * alinéas [0013], [0184], [0161], [0251], [0270], [0123]; revendication 1 * ----- | 1-15 | INV. C12M1/107 C12M1/00 C12P5/02 C12M1/36 |
| X | WO 2011/010275 A1 (ONDEO IND SOLUTIONS [FR]; DELPORTE CLAUDE [FR]; MAILLARD SARAH [FR]) 27 janvier 2011 (2011-01-27) * revendication 1; figures 1,3 * ----- | 1,12 | |
| A | EP 0 135 486 A1 (SCA DEVELOPMENT AB [SE]) 27 mars 1985 (1985-03-27) * revendication 1; figure 1 * ----- | 1-15 | |

| | | | DOMAINES TECHNIQUES RECHERCHES (IPC) |
| | | | C12M C12P |

Le présent rapport a été établi pour toutes les revendications

| Lieu de la recherche Berlin | Date d'achèvement de la recherche 4 octobre 2013 | Examinateur Jones, Laura |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

                              

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 13 16 6282

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-10-2013

| Document brevet cité au rapport de recherche | | Date de publication | Membre(s) de la famille de brevet(s) | | Date de publication |
|---|---|---|---|---|---|
| US 2012125840 | A1 | 24-05-2012 | US | 2012125840 A1 | 24-05-2012 |
| | | | US | 2013193067 A1 | 01-08-2013 |
| WO 2011010275 | A1 | 27-01-2011 | EP | 2456724 A1 | 30-05-2012 |
| | | | FR | 2948355 A1 | 28-01-2011 |
| | | | WO | 2011010275 A1 | 27-01-2011 |
| EP 0135486 | A1 | 27-03-1985 | AU | 556436 B2 | 06-11-1986 |
| | | | AU | 3119384 A | 14-02-1985 |
| | | | CA | 1236596 A1 | 10-05-1988 |
| | | | DE | 135486 T1 | 05-06-1985 |
| | | | DE | 3466670 D1 | 12-11-1987 |
| | | | EP | 0135486 A1 | 27-03-1985 |
| | | | FI | 843133 A | 11-02-1985 |
| | | | JP | S6058296 A | 04-04-1985 |
| | | | JP | S6332519 B2 | 30-06-1988 |
| | | | NZ | 209163 A | 30-04-1987 |
| | | | SE | 437257 B | 18-02-1985 |
| | | | US | 4659471 A | 21-04-1987 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2920761

- EP 2207873 A